# EUROPEAN PATENT APPLICATION

(11) **EP 4 394 096 A1**
(43) Date of publication of application: **03.07.2024**
(21) Application number: 22711857.7
(22) Date of filing: 24.02.2022
(51) Int. Cl.: C40B 50/06, C12N 15/10

(54) **METHOD FOR CONSTRUCTING DNA NEXT-GENERATION SEQUENCING LIBRARY**

(30) Priority: 25.08.2021 CN 202110981921
(71) Applicant: Daan Gene Co., Ltd., Guangzhou, Guangdong 510665 (CN)
(72) Inventor: LIANG, Zhikun, Guangzhou Guangdong 510665 (CN); JIANG, Xiwen, Guangzhou Guangdong 510665 (CN); WEN, Huimin, Guangzhou Guangdong 510665 (CN); WU, Yilan, Guangzhou Guangdong 510665 (CN)
(74) Representative: Handsome I.P. Ltd
(86) International application number: PCT/CN2022/077613
(87) International publication number: WO 2023/024464

(57) **Abstract**

Disclosed is a preparation method for a DNA next-generation sequencing library, including steps: digestion, end repair, and A-tailing of genomic DNA; adapter ligation of DNA fragments; bead purification of product after adapter ligation; PCR amplification of DNA fragments; and selection and purification of PCR product fragments. The preparation method for the DNA next-generation sequencing library includes steps: fractionating by VVN and T7 through a single-step reaction process with double digestion and end repair, blunting a 5'-overhang under the polymerization action of a Taq DNA polymerase, adding an A (adenine) to a 3'-end, and achieving the preparation of the DNA next-generation sequencing library under an integrated single-step reaction. After the single-step reaction ends, bead purification isn't required, so that the preparation process is simple. In the preparation process, there is no preference for two restriction enzymes, which achieves the sequencing of target fragments.

## Description

This application is based upon and claims priority to Chinese Patent Application No. 202110981921.0, filed to China National Intellectual Property Administration on August 25, 2021 and entitled "PREPARATION METHOD FOR DNA NEXT-GENERATION SEQUENCING LIBRARY", the content of which is incorporated herein by reference in its entirety.

### TECHNICAL FIELD

The present application relates to the field of biotechnology, in particular to a preparation method for a DNA next-generation sequencing library.

### BACKGROUND

Gene sequencers not only are the origin of sequencing industrial chains, but are also key links, which serve for the most basic support for sequencing at middle and lower reaches, and meanwhile, the gene sequencers are sections with the highest barrier, which are at the top of gene sequencing industrial value chains.

At present, the major upstream suppliers of sequencing are from overseas, among which competitions are extremely intense. Applied Biosystem Company launched a first automatic sequencer for commercial use in 1986, sounding the clarion call to seize market shares. The market competition of the sequencers moved towards relaxation from 1986 to 2005, with ABI Company grabbing the market shares basically; and the whole market shares are relatively small, with the market remaining immature. 454 Life Sciences Company launched 454 GS 20 sequencers in 2005; Illumina acquired Solexa in 2006; and ABI Company launched Solid sequencing platforms in 2006. Next-generation sequencers gradually accessed to markets, further improving on the demand quantity and expanding the overall market capacity, while seizing the market shares quickly. In the following decade, major giants scrambled to complete mergers and acquisitions of outstanding enterprises, improved sequencing technologies gradually, and launched new products continuously, leading to the rapid expansion of market scales.

The next-generation sequencers have advantages of high throughput, low costs, stable and reliable data, and the like, which have made a primary contribution to the field of sequencers. Over the last decade or so, a market pattern has been gradually established with Illumina as the bellwether and Thermo Fisher and Roche as dominant suppliers. In recent years, Beijing Genomics Institute (BGI) has also launched self-developed gene sequencers. As sequencer technologies are extremely high in barriers, and market concentration rates are extremely high, domestic sequencer markets are dominated by the above companies, in which the Illumina Company leads all suppliers in its products; and it is unanimous that Illumina has occupied 70% to 80% of global market shares.

Different sequencing platforms will differ in technical details, however, there are several common points for them as follows:
1. Library preparation: all next-generation sequencing platforms require a gene library which contains adapter sequences customized with extension or ligation.
2. Sequencer: each library fragment undergoes an amplification on solid surfaces with well-matched sequences in libraries as templates under the action of DNA ligation factors with covalent adsorptions, which generates a lot of DNA clusters; and each DNA cluster originates from one library fragment, and each gene cluster achieves an effect as independent sequencing reactions.
3. Data output: each instrument will provide source data after sequencing ends. The original data is a set of DNA sequences formed in each gene cluster.

The preparation of the sequencing gene libraries is an initial step, and the library is an aggregate containing all gene sequences to be sequenced. Adapters are added on two ends of all short sequences obtained after fractionation, so that the sequences to be sequenced are prepared into an aggregate of sequence fragments having many identical paired-end sequences, but differing in inner sequences.

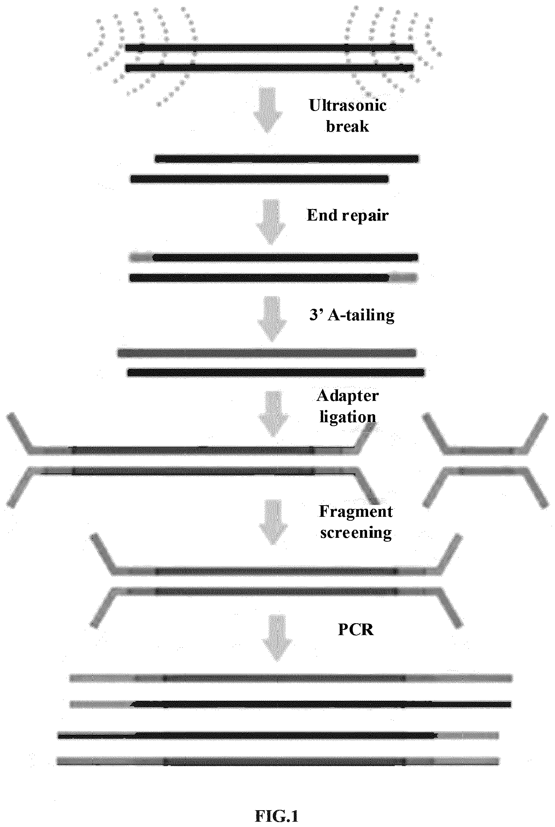

Taking an illumina platform as an example, with reference to FIG. 1, the conventional library preparation process includes the following steps:
(1) The sequences or genomes to be sequenced are treated with mechanical breaks (ultrasonic breaks) or methods in enzymology, and fractionated DNA sequences are obtained, with each fragment ranging from approximately 200 bp to 300 bp.
(2) It is very likely that the fractionated sequences are those with overhangs, which are not in complete pairing states, and thus, it is required to blunt with enzymes.
(3) A-tailings (adenine) are added on two blunt ends of the sequences with the enzymes respectively.
(4) With the A-tailings in the previous step, the adapters made by the illumina Company can be added to the two ends of the sequences easily.
(5) After the above steps are completed, making steps ends.

In the above library preparation process with the conventional steps, it is necessary to increase the quantity of sequences to be sequenced to improve the accuracy of sequencing, thereby facilitating completing a subsequent sequence assembly step. Finally, it is also necessary to increase the quantity of the sequences in gene libraries by a PCR amplification operation; and as adapters are ligated on two ends of each sequence, the pairing of design primers with the adapters only needs to be ensured.

Next-generation sequencing library preparation technologies include steps: fractionating long fragments, e.g., mechanical breaks and breaks in enzymic methods; conducting end repair on the fragments with T4 DNA polymerases, blunting, phosphorylating 5'-ends by T4 PNK, and adding 3' A-tails (adenine) with Taq DNA polymerases. However, during the preparation of the existing next-generation sequencing library, fractionation, end repair, and A-tailing fail to be implemented in an integrated process, or even though the above steps can be implemented in the integrated process, there are defects, such as increase in template quantity, and lack of internal standards for the quality control of library preparation and sequencing links, and the correction of results. Therefore, it is of practical value for developing a simplified library preparation method for DNA next-generation sequencing to achieve the quality control to the library preparation and the sequencing links and the correction of the results.

### SUMMARY

Based on the above problems, the problem to be solved in the present application is to provide a preparation method for a DNA next-generation sequencing library with a single-step reaction integrated with double digestion and end repair.

The technical solution of the present application is as follow:
The preparation method for the DNA next-generation sequencing library includes the following steps:
S1, digestion, end repair, and A-tailing of genomic DNA: conducting double digestion on the genomic DNA with two common restriction enzymes to obtain restriction fragments; and then, conducting the end repair on the restriction fragments and 3' A-tailing of the DNA with a polymerase to obtain a product with an A-tailing for the DNA;
S2, ligating the product with the A-tailing for the DNA with an adapter with a 5' T-overhang of the DNA with a ligase to obtain DNA fragments in an "adapter-DNA insertion fragment-adapter" form;
S3, recovering the DNA fragments by a primary purification treatment to obtain target DNA fragments;
S4, conducting a PCR amplification with the target DNA fragments as a template, and enriching the target DNA fragments to obtain the DNA next-generation sequencing library by preparation.

In the present application, the preparation method for the DNA next-generation sequencing library includes steps: fractionating by VVN and T7 through a single-step reaction process with double digestion and end repair based on a library preparation process of a high-throughput sequencing technology, blunting a 5'-overhang under a polymerization action of a Taq DNA polymerase, adding a 3' A-tail (adenine), and achieving the preparation of the DNA next-generation sequencing library under an integrated single-step reaction. A-tailing is integrated into the single-step reaction, which may also be compatible with a ligation reaction system. After the single-step reaction ends, bead purification isn't required, which simplifies a preparation process; and in the preparation process, there is no preference for the two restriction enzymes, which achieves a long-fragment sequencing.

### BRIEF DESCRIPTION OF THE DRAWINGS

FIG. 1 is a process flowchart illustrating the conventional DNA library preparation;
FIG. 2 is a process flowchart illustrating the preparation of a DNA next-generation sequencing library in the present application;
FIG. 3 is a structural schematic diagram illustrating adapters of a DNA insertion fragment in the present application.

### DETAILED DESCRIPTION OF THE EMBODIMENTS

The preferred embodiments of the present application will be further explained with reference to the drawings.

A high-throughput sequencing technology is also referred to as a "next-generation" sequencing technology or next-generation sequencing, by which hundreds of thousands of to millions of DNA molecules may undergo sequencing in parallel at a time, and generally, reads are short. An application process of the high-throughput sequencing technology includes sample preparation, library preparation, sequencing reaction, and data analysis.

A DNA sequencing technology of the present application is used for a library preparation process of the high-throughput sequencing technology. A complete next-generation sequencing library is a set of double-stranded DNAs (or cDNAs), in which a 5'-adapter and 3'-adapter sequence of each DNA fragment is fixed, but an insertion fragment (DNA) in the middle may vary. Therefore, the preparation of the sequencing library in the present application is a process to ligate specific adapters at upstream and downstream of unknown sequences, add sequencing primers and index sequences, and enrich them with PCR (Polymerase Chain Reaction) sequentially.

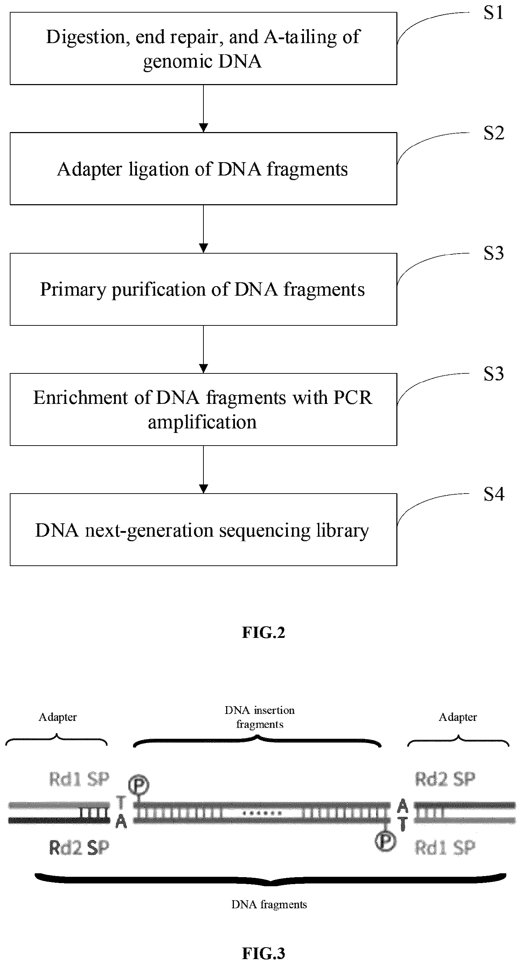

As shown in the FIG. 2, a preparation method for a DNA next-generation sequencing library of the present application includes the following process steps:
At S 1, digestion, end repair, and A-tailing of genomic DNA.

In the step, specific operation steps are as follows:
At S11, double digestion is conducted on the genomic DNA with two common restriction enzymes, and restriction fragments are obtained, where the two common restriction enzymes are VVN and T7, respectively.

At S12, the end repair of the restriction fragments and 3' adapter A-tailing (adenine) of the DNA are conducted with a polymerase, and a restriction fragment product is obtained, where the polymerase is a Taq DNA polymerase; and under the action of the polymerase, a 5'-overhang of the DNA is blunted, and an A-tail (adenine) is added at a 3' adapter of the DNA. A reaction system and a reaction condition are shown in Table 1

**Table 1 A reaction system and a reaction condition**

| Component | 1×reaction volume (µL) |
|---|---|
| Genomic DNA | X |
| Two restriction enzymes | 4 |
| Taq DNA polymerase (5 U/µL) | 1 |
| Nuclease-free water | 35-X |
| 5* reaction buffer | 10 |
| Total amount of reaction | 50 |

Where a digestion reaction of the genomic DNA happens at 37°C for 20 min to 30 min; and an A-tailing reaction happens at 65 °C for 20 min to 30 min.

At S2, adapter ligation of DNA fragments.

In the step, the product with the A tail for the DNA, obtained in the step S1, is ligated with the adapter with a 5' T-overhang of the DNA with a ligase, such as a T4 DNA ligase, and DNA fragments in an "adapter-DNA insertion fragment-adapter" form are obtained, as shown in the FIG. 3.

A reaction system and a reaction condition of the adapter ligation process of the DNA fragments are shown in Table 2.

**Table 2 A reaction system and a reaction condition**

| Component | 1×reaction volume (µL) |
|---|---|
| Product of the previous step | 50 |
| T4 DNA ligase | 5 |
| Adapter | 5 |
| 4* reaction buffer | 20 |
| Total amount of reaction | 80 |

Where a reaction of the adapter ligation happens at 20 °C for 20 min to 30 min.

At S3, primary purification of the DNA fragments.

In the step, with the primary purification treatment, the DNA fragments are recovered, and target DNA fragments are obtained. Specific process steps are as follows:
At S31, after the adapter ligation reaction for the 5' A-tailing of the DNA ends, the DNA fragments are placed in a centrifugal tube, e.g., a PCR tube; 72 µL of DNA beads (0.9X) are added, a solution is blown away or shaken at a low speed, so that the beads are mixed with the DNA fragments; liquid drops on a tube wall and a cap of the centrifugal tube are shaken off by centrifuging at the low speed; the beads are dispersed in the solution uniformly for reacting at a room temperature for 5 min, while flicking a bottom of the centrifugal tube; and if liquid is shaken to the tube wall of the centrifugal tube, the centrifugal tube is shaken lightly or centrifuged at the low speed, so that the liquid is at the bottom of the centrifugal tube.

At S32, the EP tube is inserted into a tube hole of a magnetic grate, and after the DNA fragments are adsorbed by the beads completely and the solution becomes clarified, the solution in the centrifugal tube is pipetted with a middle pipette tip. Note that don't pipet the beads, and keep the EP tube on the magnetic grate.

At S33, 200 µL of 80 v/v% ethanol is pipetted into the EP tube, while the EP tube is turned lightly twice, with a half-turn per time, so that the beads move on the tube wall of the centrifugal tube; and when the solution is pipetted, don't pipet the beads.

At S34, the previous step S33 is repeated.

At S35, centrifugation is conducted quickly for several seconds, so that the liquid on the tube wall of the centrifugal tube is shaken to the tube bottom; and the tube is put back to the tube hole of the magnetic grate, and after the DNA fragments are adsorbed by the beads completely, the solution is pipetted.

At S36, the tube is aired at the room temperature for 3 min to evaporate the residual ethanol. In the step, note: it is necessary to observe the dry and wet degree of the beads frequently during airing; in case that beads are too wet, it is necessary to prolong air drying time; it is necessary to avoid the excessive drying of the beads; after the beads are dried, there should no water stain on surfaces; and in case of any crack on the beads, it is necessary to cover the tube and carry out the next experimental operation immediately.

At S37, 22 µL of DNA eluant is pipetted: the eluant is added on one side where the beads are added to wash the beads to the bottom of the centrifugal tube; and the solution is blown away or shaken at the low speed, so that the beads are mixed with the DNA fragments uniformly, and a reaction is performed at the room temperature for 5 min; and while waiting, a PCR reaction mixture required for the following step is prepared, and marked with Mix3, and 50 µL of solution is pipetted into each new 0.2 mL PCR tube.

At S38, the EP tube is inserted into the tube hole of the magnetic grate, and after the DNA fragments are adsorbed by the beads completely and the solution becomes clarified, 20 µL of solution is pipetted into the new PCR tube. Note that don't pipet the beads; and purified target DNA fragments are obtained.

At S4, the DNA fragments are enriched with the PCR amplification.

In the step, the PCR amplification is conducted with the recovered DNA fragments as a template, the target DNA fragments are enriched, and the DNA next-generation sequencing library is obtained by preparation.

In specific process steps, the PCR amplification treatment includes the following steps:
S41, adding a PCR enzyme mixture and a PCR primer mixture into the PCR tube, and performing a PCR amplification reaction of the DNA fragments to enrich the target DNA fragments;

In the step, a reaction system is shown in Table 3.

**Table 3 Reaction system**

| Component | Reaction volume (µL) |
|---|---|
| Ligation product | 20 |
| PCR enzyme mixture | 25 |
| PCR primer mixture | 5 |
| Total amount of reaction system | 50 |

Where during the reaction, the PCR tube is placed on a PCR sequencer, and the reaction is conducted as per the following conditions:
1) Pre-denaturation stage: at the reaction temperature of 95 °C, the reaction time is 3min;
2) Denaturation stage: at the reaction temperature of 95 °C, the reaction time is 3 min;
3) Annealing stage: at the reaction temperature of 58 °C, the reaction time is 20 s;
4) Extension stage: at the reaction temperature of 72°C, the reaction time is 20 s;
5) Final extension stage: at the reaction temperature of 72°C, the reaction time is 5 min;
6) Storage stage: the reaction temperature is 4°C;

Wherein respectively executing 10 to 14 cycles at the stages 2) to 4); and executing once respectively at the stages 1) and 5).

S42, after the PCR amplification reaction ends, recovering a PCR amplification product, and selecting the DNA fragments of the PCR amplification product for a secondary purification treatment to obtain a DNA next-generation sequencing library.

In the step, specific process steps are as follows:
At S421, after the reaction ends, water is added to 100 µL, 80 µL of DNA beads (0.8X) are added, and the solution is blown away or shaken at the low speed, so that the beads are mixed with the PCR amplification product uniformly; the liquid drops on the tube wall and the cap of the centrifugal tube are shaken off by centrifuging at the low speed; the beads are dispersed in the solution uniformly for reacting at the room temperature for 5 min, while the bottom of the centrifugal tube is flicked; and if the liquid is shaken to the tube wall of the centrifugal tube, the centrifugal tube is shaken lightly or centrifuged at the low speed, so that the liquid is at the bottom of the centrifugal tube.

At S422, the EP tube is inserted into the tube hole of the magnetic grate, and after the PCR amplification product is adsorbed by the beads completely and the solution becomes clarified, the solution is transferred to a low adsorption tube containing 40 µL of DNA beads (0.4X) with the middle pipette tip; the solution is blown away or shaken at the low speed, so that the beads are mixed with the PCR amplification product; the liquid drops on the tube wall and the cap of the centrifugal tube are shaken off by centrifuging at the low speed; the beads are dispersed in the solution uniformly for reacting at the room temperature for 5 min; and if the liquid is shaken to the tube wall of the centrifugal tube, the centrifugal tube is shaken lightly or centrifuged at the low speed, so that the liquid is at the bottom of the centrifugal tube. Note that don't pipet the beads, and keep the EP tube on the magnetic grate.

At S423, the EP tube is inserted into the tube hole of the magnetic grate, and after the PCR amplification product is adsorbed by the beads completely and the solution becomes clarified, a supernatant is removed; and 200 µL of 80 v/v% ethanol is pipetted into the EP tube, while the EP tube is turned lightly twice, with a half-turn per time, so that the beads move on the tube wall of the centrifugal tube; and when the solution is pipetted, don't pipet the beads.

At S424, the previous step S423 is repeated.

At S425, centrifugation is conducted quickly for several seconds, so that the liquid on the tube wall of the centrifugal tube is shaken to the tube bottom; and the tube is put back to the tube hole of the magnetic grate, and after the PCR amplification product is adsorbed by the beads completely, the solution is pipetted.

At S426, the tube is aired at the room temperature for 3 min to evaporate the residual ethanol. In the step, note: it is necessary to observe the dry and wet degree of the beads frequently during airing; in case that beads are too wet, air drying time should be prolonged; it is necessary to avoid the excessive drying of the beads; after the beads are dried, there should no water stain on surfaces; and in case of any crack on the beads, it is necessary to cover the tube and carry out the next experimental operation immediately.

At S427, 22 µL of DNA eluant is pipetted: the eluant is added on one side where the beads are added to wash the beads to the bottom of the centrifugal tube; and the solution is blown away or shaken at the low speed repeatedly, so that the beads are mixed with the PCR amplification product uniformly, and the reaction is conducted at the room temperature for 5 min.

At S428, the EP tube is inserted into the tube hole of the magnetic grate, and after the PCR amplification product is adsorbed by the beads completely and the solution becomes clarified, 20 µL of solution is pipetted into the new EP tube (note that don't pipet the beads), and the DNA next-generation sequencing library is obtained.

It should understand that the above detailed description of the preferred embodiment of the present application isn't construed as a limitation to the scope of patent protection of the present application. The scope of patent protection of the present application should be based on the claims.

## Claims

1. A preparation method for a DNA next-generation sequencing library, comprising the following steps:
S1, digestion, end repair, and A-tailing of genomic DNA: conducting double digestion on the genomic DNA with two common restriction enzymes to obtain restriction fragments; and then, conducting the end repair on the restriction fragments and 3' A-tailing of the DNA with a polymerase to obtain a product with an A-tailing for the DNA;
S2, ligating the product with the A-tailing for the DNA with an adapter with a 5' T-overhang of the DNA with a ligase to obtain DNA fragments in an "adapter-DNA insertion fragment-adapter" form;
S3,, recovering the DNA fragments by a primary purification treatment to obtain target DNA fragments;
S4, conducting a PCR amplification with the target DNA fragments as a template, and enriching the target DNA fragments to obtain the DNA next-generation sequencing library by preparation.

2. The preparation method according to claim 1, wherein in the step S1, the two restriction enzymes are VVN and T7, respectively.

3. The preparation method according to claim 1, wherein in the step S1, the polymerase is Taq DNA polymerases.

4. The preparation method according to claim 1, wherein in the step S1, a digestion reaction of the genomic DNA happens at 37°C for 20 min to 30 min; and an A-tailing reaction happens at 65 °C for 20 min to 30 min.

5. The preparation method according to claim 1, wherein in the step S2, the ligase is a T4 DNA ligase.

6. The preparation method according to claim 1, wherein in the step S2, a reaction of the adapter ligation happens at 20°C for 20 min to 30 min.

7. The preparation method according to claim 1, wherein in the step S3, the primary purification treatment comprises the following steps: S31, after the adapter ligation reaction ends, placing the DNA fragments in a centrifugal tube, adding 72 µL of DNA beads, blowing away or shaking a solution at a low speed to make the beads mixed with the DNA fragments uniformly; shaking off liquid drops on a tube wall and a cap of the centrifugal tube by centrifuging at the low speed; and making the beads disperse in the solution uniformly for reacting at a room temperature for 5 min;
S32, inserting the EP tube into a tube hole of a magnetic grate, and after the DNA fragments are adsorbed by the beads completely and the solution becomes clarified, pipetting the solution in the centrifugal tube;
S33, pipetting 200 µL of 80 v/v% ethanol into the EP tube, while lightly turning the EP tube twice, and taking a half-turn per time to make the beads move on the tube wall of the centrifugal tube;
S34, repeating the previous step S33;
S35, centrifuging quickly for several seconds, so that liquid is shaken to the tube wall of the centrifugal tube to a tube bottom; and after the DNA fragments are adsorbed by the beads completely, pipetting the solution into the centrifugal tube;
S36, airing at the room temperature for 3 min for evaporating the residual ethanol;
S37, pipetting 22 µL of DNA eluant: adding the eluant on one side where the beads are added to wash the beads to the bottom of the centrifugal tube; and repeatedly blowing away or shaking the solution at the low speed to make the beads mixed with the DNA fragments uniformly, and reacting at the room temperature for 5 min;
S38, inserting the EP tube into the tube hole of the magnetic grate, and after the DNA fragments are adsorbed by the beads completely and the solution becomes clarified, pipetting 20 µL of solution into a new PCR tube to obtain purified target DNA fragments.

8. The preparation method according to claim 7, wherein the step S31 further comprises steps: while reacting at the room temperature for 5 min, flicking the bottom of the centrifugal tube, and making the beads distribute uniformly; and if the liquid is shaken to the tube wall of the centrifugal tube, shaking the centrifugal tube lightly or centrifuging it at the low speed, so that the liquid is at the bottom of the centrifugal tube.

9. The preparation method according to claim 7, wherein in the step S31, a concentration of the added 72 µL of DNA beads is 0.9X.

10. The preparation method according to claim 1, wherein in the step S4, the PCR amplification treatment comprises the following steps:
S41, adding a PCR enzyme mixture and a PCR primer mixture into the PCR tube, and performing a PCR amplification reaction of the DNA fragments to enrich the target DNA fragments;
S42, after the PCR amplification reaction ends, and recovering a PCR amplification product, selecting the DNA fragments of the PCR amplification product for a secondary purification treatment to obtain a DNA next-generation sequencing library.

11. The preparation method according to claim 10, wherein in the step S41, in the PCR amplification reaction, reaction temperature and reaction time are as follows according to a reaction stage:
1) Pre-denaturation stage: at the reaction temperature of 95 °C, the reaction time is 3 min;
2) Denaturation stage: at the reaction temperature of 95°C, the reaction time is 20 s;
3) Annealing stage: at the reaction temperature of 58°C, the reaction time is 20 s;
4) Extension stage: at the reaction temperature of 72°C, the reaction time is 20 s;
5) Final extension stage: at the reaction temperature of 72°C, the reaction time is 5 min;
6) Storage stage: the reaction temperature is 4°C;
Wherein respectively executing 10 to 14 cycles at the stages 2) to 4); and executing once respectively at the stages 1) and 5).

12. The preparation method according to claim 10, wherein in the step S42, after the PCR amplification reaction ends, it further comprises the following steps: selecting the DNA fragments and conducting secondary purification:
S421, after the reaction ends, adding water to 100 µL, then, adding 80 µL of DNA beads, blowing away or shaking the solution at the low speed to make the beads mixed with the PCR amplification product uniformly; shaking off the liquid drops on the tube wall and the cap of the centrifugal tube by centrifuging at the low speed; and making the beads disperse in the solution uniformly for reacting at the room temperature for 5 min, while flicking the bottom of the centrifugal tube;
S422, inserting the EP tube into the tube hole of the magnetic grate, and after the PCR amplification product is adsorbed by the beads completely and the solution becomes clarified, transferring the solution to a low adsorption tube containing 40 µL of DNA beads; blowing away or shaking the solution at the low speed to make the beads mixed with the PCR amplification product uniformly; shaking off the liquid drops on the tube wall and the cap of the centrifugal tube by centrifuging at the low speed; and making the beads disperse in the solution uniformly for reacting at the room temperature for 5 min;
S423, inserting the EP tube into the tube hole of the magnetic grate, and after the PCR amplification product is adsorbed by the beads completely and the solution becomes clarified, removing a supernatant; and pipetting 200 µL of 80 v/v% ethanol into the EP tube, while lightly turning the EP tube twice, and taking a half-turn per time to make the beads move on the tube wall of the centrifugal tube;
S424, repeating the previous step S423;
S425, centrifuging quickly for several seconds, so that the liquid is shaken to the tube wall of the centrifugal tube to a tube bottom; and after the PCR amplification product is adsorbed by the beads completely, pipetting the solution into the centrifugal tube;
S426, airing at the room temperature for 3 min for evaporating the residual ethanol;
S427, pipetting 22 µL of DNA eluant: adding the eluant on one side where the beads are added to wash the beads to the bottom of the centrifugal tube; and blowing away or shaking the solution at the low speed repeatedly to make the beads mixed with the PCR amplification product uniformly, and reacting at the room temperature for 5 min;
S428, inserting the EP tube into the tube hole of the magnetic grate, and after the PCR amplification product is adsorbed by the beads completely and the solution becomes clarified, pipetting 20 µL of solution into the new EP tube to obtain the DNA next-generation sequencing library.

13. The preparation method according to claim 12, wherein in the step S421, a concentration of the added 80 µL of DNA beads is 0.8X.

14. The preparation method according to claim 12, wherein in the step S422, a concentration of the added 40 µL of DNA beads is 0.4X.

15. The preparation method according to claim 12, wherein the step S422 further comprises steps: while reacting at the room temperature for 5 min, flicking the bottom of the centrifugal tube, and making the beads distribute uniformly; and if the liquid is shaken to the tube wall of the centrifugal tube, shaking the centrifugal tube lightly or centrifuging it at the low speed, so that the liquid is at the bottom of the centrifugal tube.

16. The preparation method according to claim 10, wherein in the step S41, a volume of a ligation product in the PCR tube is 20 µL.

17. The preparation method according to claim 10, wherein in the step S41, a volume of the PCR enzyme mixture is 25 µL.

18. The preparation method according to claim 10, wherein in the step S41, a volume of the PCR primer mixture is 5 µL.

19. The preparation method according to claim 1, wherein the Taq DNA polymerase is metered as 5 U/µL.

20. The preparation method according to claim 1, wherein reaction volumes of the two restriction enzymes are 4 µL.
